Europäisches Patentamt

European Patent Office

Office européen des brevets

(19)

(11) Publication number: **0 321 988**
**A1**

# EUROPEAN PATENT APPLICATION

(21) Application number: 88121534.7

(22) Date of filing: 22.12.88

(51) Int. Cl.4: **G01N 33/68** , **G01N 33/535**

(30) Priority: 23.12.87 PL 269671

(43) Date of publication of application:
28.06.89 Bulletin 89/26

(84) Designated Contracting States:
DE FR GB

(71) Applicant: PRZEDSIEBIORSTWO
ZAGRANICZNE "PLASTOMED"
ul. Daniszewska 4
PL-03-230 Warszawa(PL)

(72) Inventor: Chorzelski, Tadeusz
ul. Okinskiego 1/44
PL-02-115 Warszawa(PL)
Inventor: Sulej, Jadwiga
ul. Kamzimierzowska 71/75 m. 45
PL-02-518 Warszawa(PL)
Inventor: Krainska-Wojcik, Teresa
ul. Strubiczow 4a M. 18
PL-02-246 Warszawa(PL)
Inventor: Roman, Malgorzata
ul. Pulku Baszta 2 m. 11
PL-02-649 Warszawa(PL)

(74) Representative: Martin, Jean-Jacques et al
Cabinet REGIMBEAU 26, Avenue Kléber
F-75116 Paris(FR)

(54) Gluten enteropathy test.

(57) The present invention solves the problem of quick and non-invasive screening of gluten enteropathy in coeliac desease and in dermatitis and herpetiformis.

A test contains an immunological conjugate constituting the anti human IgA antibodies conjugated with peroxidase, and a substance, preferably 3,3' - diaminobenzidine tetrachloride, causing a change of staining of fibres at places where the conjugate is bounded, the said antibodies being dripped upon the biopsy specimens with the priorly applied patient's serum and control serums.

Furthermore, the test contains a buffered physiological salt solution as a conjugate diluting agent and as a washing agent, a buffered Tris-HCl as a diluting agent of the substance causing a change of staining, and a buffered glycerin as a terminating agent.

## Gluten enteropathy test

The subject of the present invention is a test enabling the quick and non-invasive screening of gluten enteropathy in coeliac desease and in dermatitis herpetiformis.

A method for the screening of gluten enteropathy, as recommended by the European Society for Pediatric Gastroenterology and Nutrition, consists in multiple intestinal biopsies. Small intestine biopsies are taken and the fibres collected are microscopically examined. A patient, most frequently an infant who is passing from natural feeding via mother's milk to artificial nutrition, is to undergo at least three small intestine biopsies. Such a biopsy is applied in preliminary diagnosis before recommending the adherence to the gluten free diet, as a control means in the course of the diet, and also in gluten post challenge for final confirmation of the diagnosis. The invasive diagnosing of gluten enteropathy in coeliac desease is time-consuming, as it lasts for a few years before the said diagnosis is confirmed or rejected. Moreover, the biopsy itself appears unpleasant to the patient, and it requires attendance of the specialised medical personnel. It is also labour- and time-consuming to make preparations from the small intestine biopsy specimens.

A discovery of the immunological marker of gluten enteropathy made it possible to substitute intestinal biopsies by an immunological test, and to shorten the time of diagnosis of coeliac desease from a few years to a few months. The said marker can be the Iga anti endomysial antibodies of the smooth muscle fibres - Iga-EmA. They appear in the serum of patients in all the cases of coeliac desease in an acute stage, to disappear when gluten is eliminated from the diet, and to keep appearing at patients not adhering to the gluten free diet.

The said antibodies appear again within the period of two weeks up to three months since the gluten challenge has been commenced.

The test for the screening of gluten enteropathy according to the present invention contains an immunological conjugate constituting the anti human Iga antibodies conjugated with peroxidase, and a substance, preferably 3,3′ -diaminobenzidine tetrachloride, causing a change of staining of fibres at places where conjugate is bounded, the said antibodies being dripped upon the biopsy specimens of fibres with the priorly applied patient's serum and control serums thereon. The said test further contains a conjugate as a diluting agent, a buffered physiological salt solution as a washing agent, a buffered Tris-HCL as an agent diluting the substance which alters the staining, and a buffered glycerin as a terminating agent.

The test according to the present invention is easy to be made and relatively quick, as it enables detection of the Iga-EmA antibodies in the course of two or three hours.

The preparations obtained are durable, and the results of staining are examined with the aid of a plain light microscope being available in each laboratory.

Example.

The test for the screening of gluten enteropathy contains an immunological conjugate constituting the anti human IgA antibodies conjugated with peroxidase, and 3,3′ - diaminobenzidine tetrachloride being the substance which causes a change of staining of fibres at places where conjugate is bounded. The said test further contains a conjugate as a diluting agent, a buffered physiological salt solution as a washing agent, a buffered Tris-HCL as an agent which dilutes the substance altering the staining, and a buffered glycerin as a terminating agent.

For the sake of screening of gluten enteropathy with the aid of the test as per the present invention, after the biopsy specimens of fibres are placed upon the slide mi croscopicals, the diluted patient's serum is dripped thereon together with the positive or negative control serums. The biopsy specimens of fibres together with serums are subjected to incubation into a humid chamber at a room temperature in the course of 30 minutes. Then they are washed twice into a buffered physiological salt solution in the course of 10 minutes, and the excess of fluid is removed. Upon the biopsy specimens of fibres being prepared in this way, a peroxidase conjugate being diluted with a buffered physiological salt solution is dripped. Afterwards, the said biopsy specimens are in turn subjected to incubation and washing, when the excess of fluid is removed in the above stated manner. Then 3,3′ - diaminobenzidine tetrachloride is dripped upon the said biopsy specimens, being diluted into a buffered Tris-HCL of 7,6 basic pH with addition of hydrogen peroxide of concentration not exeeding 0,03 %, being the substance which causes a change of staining of fibres at places where peroxidase conjugate is bounded. On being dripped upon, the biopsy specimens of fibres are subjected to incubation into a humid chamber at a room temperature in the course of 10 minutes, washed twice in distilled water in the course of 10 minutes, when the excess of fluid is removed, and terminated into a buffered glycerin. Then the biopsy specimens of fibres being prepared in this manner are examined under a light microscope. In the case then the anti endomysial antibodies of the smooth muscle fibres - Iga-EmA are detected in the patient's serum, there occurs a characteristic brown staining of the endomysium of the smooth muscle fibres. Into the biopsy specimens of fibres with negative control serums, such a staining of endomysium is missing.

With the aid of the test as per the present invention, various qualitative and quantitative tests can be performed. In a qualitative test, it is being pre-checked whether there occur any anti endomysial antibodies of the smooth muscle fibres - IgA-EmA into the serums examined. In a quantitative test, the level of the IgA-EmA antibodies into the serum is being specified.

## Claims

A gluten enteropathy test, characterised in that it contains an immunological conjugate constituting the anti human IgA antibodies conjugated with peroxidase, and a substance, preferably 3,3' - diaminobenzidine tetrachloride, causing a change of staining of fibres where the conjugate is bounded, the said antibodies being dripped upon the biopsy specimens of fibres with the priorly applied patient's serum and control serums thereon, whereas it contains a buffered physiological salt solution as a conjugate diluted agent and as a washing agent, a buffered Tris-HCL as a diluting agent of the substance causing a change of staining, and a buffered glycerin as a terminating agent.

| DOCUMENTS CONSIDERED TO BE RELEVANT | | | EP 88121534.7 |
|---|---|---|---|
| Category | Citation of document with indication, where appropriate, of relevant passages | Relevant to claim | CLASSIFICATION OF THE APPLICATION (Int. Cl.4) |
| P,X | CAS<br><br>T.KRAINSKA et al. "Detection of specific marker for gluten-dependent enteropathy by immunoenzymic method. Comparison of immuno-peroxidas and immunofluorescent method"<br>Abstract-no. 109-209 260<br><br>& Przegl. Dermatol. 88, vol. 75, no. 2, page 123-8<br><br>-- | 1 | G 01 N 33/68<br><br>G 01 N 33/535 |
| A | MEDLINE<br><br>KP ETERMAN et al. "Failure to detect specific gluten antigenes associated with the immune aggregates in the skin in dermatitis herpetiformis"<br>Abstract-no. 78101870<br><br>& ARCHIVES FOR DERMATOLOGICAL RESEARCH, December 27, 1977, vol. 260, no. 3, page 247-52<br><br>-- | 1 | |
| A | CHEMICAL ABSTRACTS, vol. 89, no. 23, December 4, 1978, Columbus, Ohio, USA<br><br>Z.M.FALCHUK et al. "Organ culture model of glutensensitive entero-pathy"<br>Abstract-no. 194 938t<br><br>& Perspect. Coeliac Dis., Proc. Symp., 3rd, 1977 (Pub. 1978), pages 65-73<br><br>---- | 1 | **TECHNICAL FIELDS SEARCHED (Int. Cl.4)**<br><br>G 01 N 33/00 |

The present search report has been drawn up for all claims

| Place of search | Date of completion of the search | Examiner |
|---|---|---|
| VIENNA | 03-03-1989 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82